(19) Europäisches Patentamt
European Patent Office
Office européen des brevets

(11) EP 3 348 253 B1

(12) # EUROPEAN PATENT SPECIFICATION

(45) Date of publication and mention of the grant of the patent:
**17.07.2019 Bulletin 2019/29**

(51) Int Cl.:
*A61K 8/365* (2006.01) *A61K 8/44* (2006.01)
*A61Q 11/00* (2006.01) *A61K 8/67* (2006.01)

(21) Application number: **17382005.1**

(22) Date of filing: **11.01.2017**

(54) **LOW-ALCOHOL ORAL CARE COMPOSITIONS COMPRISING ETHYL LAUROYL ARGINATE**

ALKOHOLARME MUNDPFLEGEZUSAMMENSETZUNGEN MIT ETHYLLAUROYLARGINAT

COMPOSITIONS DE SOIN BUCCAL FAIBLEMENT ALCOOLISÉES COMPRENANT DE L'ÉTHYL LAUROYL ARGINATE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**18.07.2018 Bulletin 2018/29**

(73) Proprietor: **Lacer, S.A.**
**08025 Barcelona (ES)**

(72) Inventors:
- **MATA MOLINER, Montserrat**
  **E-08290 Cerdanyola del Vallès, Barcelona (ES)**
- **DRIÉGUEZ GARCÍA, Josefa**
  **E-08290 Cerdanyola del Vallès, Barcelona (ES)**
- **FERRÀ DOMINGO, Lourdes**
  **E-08290 Cerdanyola del Vallès, Barcelona (ES)**

(74) Representative: **ABG Intellectual Property, S.L.**
**Avenida de Burgos, 16D**
**Edificio Euromor**
**28036 Madrid (ES)**

(56) References cited:
**FR-A1- 3 031 679**
**US-A1- 2004 258 632**
**US-A1- 2008 241 117**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

**Description**

## FIELD OF THE INVENTION

The present invention belongs to the field of oral care compositions and provides a stable aqueous oral rinse composition comprising ethyl lauroyl arginate hydrochloride, a source of gluconate anions providing a concentration of gluconate anions of at least 15 mM, at least 0.35% w/v of a gluconate salt, and Vitamin E or an ester thereof, wherein the composition is alcohol free or has a low content of alcohol.

## BACKGROUND OF THE INVENTION

Dental plaque or plaque bio-film is a soft deposit that forms on teeth and is implicated in the occurrence of gingivitis and other forms of periodontal disease. Several cationic agents, such as ethyl lauroyl arginate, have been found to have the clinical ability to retard the growth of bacteria and, thus, minimize plaque formation, oral infections and dental disease associated therewith (US 5,874,068 and US 2010/0330136 A1).

Ethyl lauroyl arginate is generally used as its hydrochloride salt, i.e. ethyl lauroyl arginate hydrochloride, also known as ethyl-$N^{\alpha}$-dodecanoyl-L-arginate hydrochloride (CAS No. 60372-77-2) having the chemical structure depicted below:

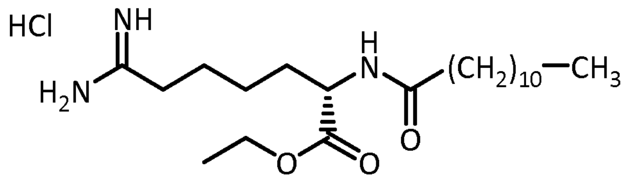

Ethyl lauroyl arginate hydrochloride is a cationic surfactant, active against a wide spectrum of activity against Gram positive and negative bacteria, algae and fungi by modifying the permeability of membranes.

Ethyl lauroyl arginate hydrochloride is listed by the FDA (Food and Drug Administration) as being a GRAS (Generally Recognized As Safe) substance.

However, it has been recognized that ethyl lauroyl arginate hydrochloride tends to lack sufficient stability to be useful in low-alcohol or alcohol-free mouth rinses (WO 2016/077464 A1 and US 5,874,068).

Some authors suggest encapsulating ethyl lauroyl arginate hydrochloride for improving its stability (US 2010/0330136 A1), others suggest using cationic-compatible inorganic particulates having a surface that is substantially inert to ethyl lauroyl arginate hydrochloride (WO 2007/011552 A2) or even derivatizing ethyl lauroyl arginate, such as by replacement of the ester moiety in ethyl lauroyl arginate hydrochloride with an amido moiety (WO 2016/077464 A1).

Nevertheless, there remains a need for stable low-alcohol or alcohol-free oral care compositions comprising ethyl lauroyl arginate hydrochloride.

## SUMMARY OF THE INVENTION

The authors of the present invention have surprisingly found that combining a gluconate salt and Vitamin E allows obtaining stable low-alcohol or alcohol-free oral rinses comprising ethyl lauroyl arginate hydrochloride.

Thus, in a first aspect the present invention relates to an aqueous oral rinse composition comprising ethyl lauroyl arginate hydrochloride, a source of gluconate anions, and Vitamin E or an ester thereof, wherein the composition comprises less than 1% w/v of a $C_1$-$C_4$ monoalcohol, and wherein the source of gluconate anions is used in an amount providing at least 15 mM of said anions in the aqueous oral rinse composition.

In a second aspect, the present invention relates to an aqueous oral rinse composition as defined in the first aspect for use in inhibiting plaque in an oral cavity.

In a third aspect, the present invention relates to the use of a combination comprising:

- a source of a gluconate anions, and
- Vitamin E or an ester thereof,

to enhance the stability of an aqueous oral rinse composition comprising ethyl lauroyl arginate hydrochloride and less than 1% w/v of a $C_1$-$C_4$ monoalcohol, wherein the source of gluconate anions is used in an amount providing at least 15 mM of said anions in the aqueous oral rinse composition.

## DESCRIPTION OF THE DRAWINGS

Figure 1 is a graph representing the evolution of the concentration of ethyl lauroyl arginate hydrochloride (in mg/ml) with time (in months) for the composition of Example 1 after storage at 25 °C (rhombus), 30 °C (squares) and 40 °C (triangles). The dotted lines represent the upper and lower limits of the concentration of ethyl lauroyl arginate hydrochloride for a composition to be considered as being stable.

Figure 2 is a graph representing the evolution of the concentration of ethyl lauroyl arginate hydrochloride (in mg/ml) with time (in months) for the composition of Comparative example 2 after storage at 25 °C (rhombus), 30 °C (squares) and 40 °C (triangles). The dotted lines represent the upper and lower limits of the concentration of ethyl lauroyl arginate hydrochloride for a composition to be considered as being stable.

Figure 3 is a graph representing the evolution of the concentration of ethyl lauroyl arginate hydrochloride (in mg/ml) with time (in months) for the composition of Comparative example 3 after storage at 25 °C (rhombus), 30 °C (squares) and 40 °C (triangles). The dotted lines represent the upper and lower limits of the concentration of ethyl lauroyl arginate hydrochloride for a composition to be considered as being stable.

## DETAILED DESCRIPTION OF THE INVENTION

Aqueous oral rinse compositions

The first aspect the present invention relates to an aqueous oral rinse composition comprising ethyl lauroyl arginate hydrochloride, a source of gluconate anions, and Vitamin E or an ester thereof, wherein the composition comprises less than 1% w/v of a $C_1$-$C_4$ monoalcohol, and wherein the source of gluconate anions is used in an amount providing at least 15 mM of said anions in the aqueous oral rinse composition.

The compositions of the present invention are oral rinse compositions. In the context of the present invention, "oral rinse" is equivalent to "mouthwash", "mouth rinse" and "mouth bath" and designate liquid compositions which are suitable for oral hygiene, such as by reducing the microbial load in the oral cavity, in particular the bacterial plaque that causes cavities, gingivitis and bad breath. When using such compositions, they are typically maintained in the mouth passively and/or swished through the teeths by contraction of the perioral muscles and/or movement of the head, and may be gargled, and finally spit out.

In the context of the present invention, when referring to a percentage of a component, unless otherwise specified, said percentage refers to the weight of a component expressed in grams (g) with respect to the total volume of the composition expressed in milliliters (ml) and multiplied by 100, i.e. it is expressed as percentage of weight by volume (% w/v).

The term "aqueous" refers to compositions comprising water, preferably at least 50% w/v, preferably at least 60% w/v, more preferably at least 70% w/v, even more preferably at least 75% w/v, still more preferably at least 80% w/v of water.

The term "ethyl lauroyl arginate hydrochloride" refers to ethyl-$N^{\alpha}$-dodecanoyl-L-arginate hydrochloride whose definition and chemical structure has been provided above.

In a particular embodiment, the ethyl lauroyl arginate hydrochloride is present in an amount of from 0.01% w/v to 1% w/v, preferably from 0.05% w/v to 0.5% w/v, even more preferably from 0.10% w/v to 0.20% w/v, still more preferably from 0.12% w/v to 0.16% w/v, still more preferably from 0.13% w/v to 0.15% w/v.

The term "source of gluconate anions" refers to compounds which comprise the anion of gluconic acid (also known as D-gluconic acid). Said sources of gluconate anions comprise gluconic acid itself and, preferably, salts of gluconic acid with an inorganic cation. Said salts may be formed by one gluconate anion and one monovalent cation (such as $Na^+$, $K^+$ and $NH_4^+$), and also salts formed by two gluconate anions and one bivalent cation (such as $Zn^{2+}$, $Ca^{2+}$, $Mg^{2+}$, $Fe^{2+}$ and $Cu^{2+}$).

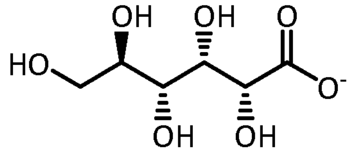

Gluconate anion

In the context of the present invention the amount of the source of gluconate anions is expressed as mM

(milimolar) concentration of gluconate anions in the aqueous oral rinse composition assuming that dissociation of the source of gluconate anions is complete (100%). Thus, 1 mM of any salt of gluconate with a monovalent cation will be considered to provide 1 mM of gluconate anions while a 1 mM of any salt of gluconate with a divalent cation will be considered to provide 2 mM of gluconate anions.

The compositions of the invention are intended for topical use in the mouth and so any salts for use in the present invention should be orally acceptable, that is, safe for topical use in the mouth in the amounts and concentrations provided. Suitable salts include salts known in the art to be pharmaceutically acceptable. Examples of such gluconate salts are sodium gluconate, potassium gluconate, zinc gluconate, calcium gluconate, magnesium gluconate, iron(II) gluconate and ammonium gluconate. Preferably, the gluconate salt is zinc gluconate, sodium gluconate or mixtures thereof.

The term "pharmaceutically acceptable" refers to molecular entities and compositions that are physiologically tolerable and do not typically produce an allergic or similar untoward reaction when administered to a human or animal. Preferably, as used herein, the term "pharmaceutically acceptable" means approved by a regulatory agency of the Federal or a state government or listed in the U.S. Pharmacopeia or other generally recognized pharmacopeia for use in animals, and more particularly in humans.

The source of gluconate anions is present in the compositions of the present invention in an amount providing a concentration of gluconate anions of at least 15 mM. Preferably, the compositions of the present invention comprise at least 17 mM of a gluconate salt, more preferably at least 19 mM, even more preferably at least 21 mM, still more preferably about 24 mM. Preferably the compositions of the present invention comprise a source of gluconate anions providing from 15 mM to 100 mM of said anions, more preferably from 17 mM to 100 mM, even more preferably from 19 mM to 100 mM, still more preferably from 21 mM to 100 mM. If several sources of gluconate anions are present, the amount of gluconate anions refers to the sum of the anions contributed by all the gluconate salts present in the composition. For example, if a composition comprises 0.34% w/v of zinc gluconate (contributing 14.92 mM) and 0.2% w/v of sodium gluconate (contributing 9.17 mM), the concentration of gluconate in said composition is 24.09 mM.

The term "Vitamin E" refers to tocopherols, tocotrienols, including their stereoisomers and mixtures thereof, such as α-tocopherol, β-tocopherol, γ-tocopherol, δ-tocopherol, α-tocotrienol, β-tocotrienol, γ-tocotrienol, δ-tocotrienol, stereoisomers thereof and mixtures thereof; preferably α-tocopherol or a stereoisomer thereof.

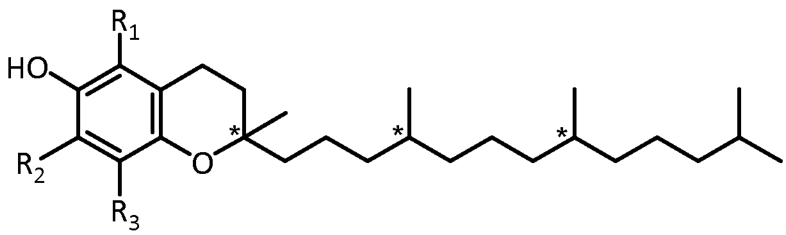

Tocopherols

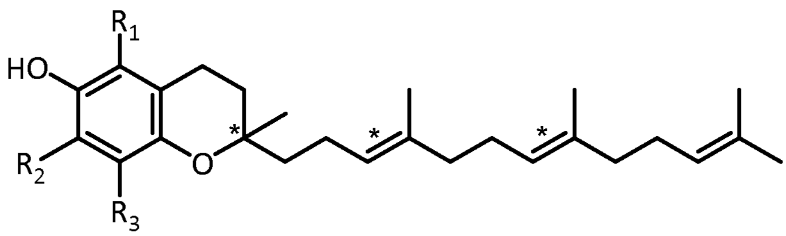

Tocotrienols

"α-Tocopherol" is also known as 2,5,7,8-tetramethyl-2-(4,8,12-trimethyltridecyl)-3,4-dihydrochromen-6-ol and has the structure shown above wherein $R_1$, $R_2$ and $R_3$ are methyl groups.

"β-Tocopherol" is also known as 2,5,8-trimethyl-2- (4,8,12-trimethyltridecyl)-3,4-dihydrochromen-6-ol and has the structure shown above wherein $R_1$ and $R_3$ are methyl groups and $R_2$ is a hydrogen atom.

"γ-Tocopherol" is also known as 2,7,8-trimethyl-2-(4,8,12-trimethyltridecyl)-3,4-dihydrochromen-6-ol and has the structure shown above wherein $R_2$ and $R_3$ are methyl groups and $R_1$ is a hydrogen atom.

"δ-Tocopherol" is also known as 2,8-dimethyl-2- (4,8,12-trimethyltridecyl)]-3,4-dihydrochromen-6-ol and has the structure shown above wherein $R_3$ is a methyl group and $R_1$ and $R_2$ is are hydrogen atoms.

The tocopherols depicted above have three chiral carbon atoms marked with * in the structure depicted above

that may be in *R* or *S* configuration, one at position 2 of the 3,4-dihydrochromene ring, one at positon 4 of the tridecyl chain and one at position 8 of the tridecyl chain. Examples of stereoisomers of any of the particular tocopherols mentioned above are the 2*R*,4*R*,8*R*-stereoisomers (or *RRR*), 2*R*,4*S*,8*R*-stereoisomers (or RSR), 2*R*,4*R*,8*S*-steroisomers (or *RRS*), 2*R*,4*S*,8*S*-steroisomers (or *RSS*), 2*S*,4*R*,8*R*-stereoisomers (or *SRR*), 2*S*,4*S*,8*R*-stereoisomers (or *SSR*), 2S,4R,8S-stereoisomers (or *SRS*) and 2S,4S,8S-stereoisomers (or *SSS*). When reference is made to tocopherol, α-tocopherol, β-tocopherol, γ-tocopherol and/or δ-tocopherol, the particular stereoisomers and mixtures of stereoisomers are also encompassed.

"α-Tocotrienol" is also known as 2,5,7,8-tetramethyl-2-(4,8,12-trimethyltrideca-3,7,11-trienyl)-3,4-dihydrochromen-6-ol and has the structure above below wherein $R_1$, $R_2$ and $R_3$ are methyl groups.

"β-Tocotrienol" is also known as 2,5,8-trimethyl-2-(4,8,12-trimethyltrideca-3,7,11-trienyl)-3,4-dihydrochromen-6-ol and has the structure shown above wherein $R_1$ and $R_3$ are methyl groups and $R_2$ is a hydrogen atom.

"γ-Tocotrienol" is also known as 2,7,8-trimethyl-2-(4,8,12-trimethyltrideca-3,7,11-trienyl)-3,4-dihydrochromen-6-ol and has the structure shown above wherein $R_2$ and $R_3$ are methyl groups and $R_1$ is a hydrogen atom.

"δ-Tocotrienol" is also known as 2,8-dimethyl-2-(4,8,12-trimethyltrideca-3,7,11-trienyl)-3,4-dihydrochromen-6-ol and has the structure shown above wherein $R_3$ is a methyl group and $R_1$ and $R_2$ is are hydrogen atoms.

The tocotrienols depicted above have one chiral carbon atoms marked with * in the structure depicted above that may be in *R* or *S* configuration, at position 2 of the 3,4-dihydrochromene ring, and two double bonds marked with * in the structure depicted above that may be in *E* or *Z* configuration, one double bond at position 3 of the tridecyl chain and double bond at position 7 of the tridecyl chain. Examples of stereoisomers of any of the above depicted tocotrienols are the 2*R*,3*E*,7*E*-stereoisomers, 2*R*,3*Z*,7*E*-stereoisomers, 2*R*,3*E*,7*Z*-steroisomers, 2*R*,3*Z*,7*Z*-steroisomers, 2*S*,3*E*,7*E*-stereoisomers, 2*S*,3*Z*,7*E*-stereoisomers, 2*S*,3*E*,7*Z*-stereoisomers and 2S,3Z,7Z-stereoisomers. When reference is made to tocotrienol, α-tocotrienol, β-tocotrienol, γ-tocotrienol and/or δ-tocotrienol, the particular stereoisomers and mixtures of stereoisomers are also encompassed.

The Vitamin E may be present as a single compound and single stereoisomer or as a mixture of different tocopherols and/or tocotrienols and/or stereoisomers thereof.

Esters of Vitamin E refer to derivatives of any of the above compounds (tocopherols, tocotrienols, stereoisomers thereof and mixtures thereof), wherein the hydroxyl group (-OH) of the phenol moiety has been replaced with an ester moiety (-OC(=O)R), i.e. tocopheryl and tocotrienyl esters, including stereoisomers thereof and mixtures thereof. Examples of esters of Vitamin E are the acetate (R is $-CH_3$) and succinate esters (R is $-CH_2CH_2COOH$), and mixtures thereof, preferably the acetate ester.

In a particular embodiment, the Vitamin E or ester thereof is tocopheryl acetate (including stereoisomers thereof) and mixtures thereof, more preferably α-tocopheryl acetate.

Preferably, the compositions of the present invention comprise from 0.01% w/v to 1% w/v of Vitamin E or an ester thereof, more preferably from 0.05% w/v to 0.5% w/v, even more preferably from 0.1% w/v to 0.3% w/v, even more preferably from 0.15% w/v to 0.25% w/v, still more preferably about 0.2% w/v.

The compositions of the invention comprise less than 1% w/v of a $C_1$-$C_4$ monoalcohol, preferably less than 0.5% w/v, more preferably less than 0.01% w/v, even more preferably less than 0.005% w/v. The compositions may also be free from a $C_1$-$C_4$ monoalcohol, i.e. no $C_1$-$C_4$ monoalcohol is present at all.

"$C_1$-$C_4$ monoalcohol" refers to a $C_1$-$C_4$ alkyl group bound to one hydroxyl group (-OH). Examples of $C_1$-$C_4$ monoalcohols are methanol, ethanol, n-propanol, isopropanol, *n*-butanol, isobutanol, *sec*-butanol, *tert*-butanol and mixtures thereof, preferably ethanol.

In another preferred embodiment, the aqueous oral rinse composition of the invention further comprises a source of $Zn^{2+}$ cations.

In the context of the present invention the amount of the source of $Zn^{2+}$ cations is expressed as mM (milimolar) concentration of $Zn^{2+}$ cations in the aqueous oral rinse composition assuming that dissociation of the source of $Zn^{2+}$ cations is complete (100%). Thus, 1 mM of any salt of $Zn^{2+}$ will be considered to provide 1 mM of $Zn^{2+}$ cations.

Preferably, the source of $Zn^{2+}$ cations are present in an amount providing a concentration of at least 1 mM of $Zn^{2+}$ cations in the aqueous oral rinse composition, preferably at least 3 mM, more preferably at least 5 mM, even more preferably at least 7 mM, still more preferably at least 7.4 mM. In another particular embodiment, the aqueous oral rinse composition of the invention comprises a source of $Zn^{2+}$ cations providing from 1 mM to 50 mM of $Zn^{2+}$ cations, more preferably from 3 mM to 50 mM, even more preferably from 5 mM to 50 mM, even more preferably from 7 mM to 50 mM, still more preferably from 7.4 mM to 50 mM.

The source of $Zn^{2+}$ can be any salt suitable for being used in aqueous oral rinse compositions, i.e. orally acceptable, preferably pharmaceutically acceptable, and having $Zn^{2+}$ as the cation, such as zinc gluconate, zinc chloride, zinc citrate, zinc lactate and zinc acetate , preferably zinc gluconate. In the particular case when zinc gluconate is present in the compositions of the invention, said salt provides both a source gluconate anions and a source of $Zn^{2+}$ cations as previously defined and is counted in both classes of compounds when assessing whether a composition falls within the scope of the present invention. Thus, a composition having 1 mM of Zinc gluconate is 2 mM in gluconate anions and 1

mM in $Zn^{2+}$ cations.

In a particular embodiment, the aqueous oral rinse composition of the invention comprises:

- from 0.10% w/v to 0.20% w/v of ethyl lauroyl arginate hydrochloride,
- a source of gluconate anions providing from 15 mM to 100 mM of said anions,
- from 0.05% w/v to 0.5% w/v of Vitamin E or an ester thereof, and
- optionally a source of $Zn^{2+}$ cations providing from 1 mM to 50 mM of said cations.

In another particular embodiment, the aqueous oral rinse composition of the invention comprises:

- from 0.12% w/v to 0.16% w/v of ethyl lauroyl arginate hydrochloride,
- a source of gluconate anions providing from 17 mM to 100 mM of said anions,
- from 0.10% w/v to 0.30% w/v of Vitamin E or an ester thereof, and
- optionally a source of $Zn^{2+}$ cations providing from 3 mM to 50 mM of said cations.

In another particular embodiment, the aqueous oral rinse composition of the invention comprises:

- from 0.12% w/v to 0.16% w/v of ethyl lauroyl arginate hydrochloride,
- a source of gluconate anions providing from 19 mM to 100 mM of said anions,
- from 0.15% w/v to 0.25% w/v of Vitamin E or an ester thereof, and
- optionally a source of $Zn^{2+}$ cations providing from 5 mM to 50 mM of said cations.

In another particular embodiment, the aqueous oral rinse composition of the invention comprises:

- from 0.13% w/v to 0.15% w/v of ethyl lauroyl arginate hydrochloride,
- a source of gluconate anions providing from 21 mM to 100 mM of said anions,
- from 0.15% w/v to 0.25% w/v of Vitamin E or an ester thereof, and
- optionally a source of $Zn^{2+}$ cations providing from 7 mM to 50 mM of said cations.

In the particular embodiments described above, preferably the source of $Zn^{2+}$ is present, and more preferably it is Zn gluconate.

In a particular embodiment, the aqueous oral rinse composition of the invention comprises:

- from 0.10% w/v to 0.20% w/v of ethyl lauroyl arginate hydrochloride,
- from 2 mM w/v to 21 mM of sodium gluconate,
- from 5 mM w/v to 50 mM of zinc gluconate, and
- from 0.05% w/v to 0.5% w/v of tocopheryl acetate,

wherein the total concentration of gluconate anions is at least 15 mM.

In another particular embodiment, the aqueous oral rinse composition of the invention comprises:

- from 0.12% w/v to 0.16% w/v of ethyl lauroyl arginate hydrochloride,
- from 4 mM to 12 mM of sodium gluconate,
- from 5 mM to 50 mM of zinc gluconate, and
- from 0.10% w/v to 0.30% w/v of tocopheryl acetate,

wherein the total concentration of gluconate anions is at least 15 mM.

In another particular embodiment, the aqueous oral rinse composition of the invention comprises:

- from 0.13% w/v to 0.15% w/v of ethyl lauroyl arginate hydrochloride,
- from 6 mM to 10 mM of sodium gluconate,
- from 5 mM to 50 mM of zinc gluconate, and
- from 0.15% w/v to 0.25% w/v of tocopheryl acetate,

wherein the total concentration of gluconate anions is at least 15 mM.

The aqueous oral rinse compositions of the invention may also comprise further conventional ingredients for oral rinses. Thus, in a preferred embodiment, the aqueous oral composition of the invention further comprises one or more ingredients selected from the group consisting of humectants, surfactants, antioxidants, buffering agents, thickeners

and flavoring agents.

The term "humectant" refers to compound which is capable of holding and retaining moisture. Examples of humectants are polyhydric alcohols such as glycerin, propylene glycol, xylitol, sorbitol and polyethylene glycol. Preferably, the humectant is selected from the group consisting of glycerin, xylitol, propylene glycol and mixtures thereof. In particular, the amount of humectant in the compositions of the invention is from 5% w/v to 20% w/v, more preferably from 10% w/v to 15% w/v.

The term "surfactant" refers to compounds that are capable of lowering the surface tension. Suitable surfactants include non-ionic, anionic, cationic and amphoteric surfactants, preferably non-ionic surfactants.

Examples of non-ionic surfactants are block copolymers such as ethylene oxide and propylene oxide block copolymers (e.g. poloxamers), ethoxylated hydrogenated castor oils, polyoxyethylene sorbitan esters (e.g. polysorbates), fatty alcohol ethoxylates, alkylphenol ethoxylates, tertiary amine oxides, tertiary phosphine oxides, dialkylsulfoxides and the like. In a particular embodiment, the non-ionic surfactant is selected from ethylene oxide and propylene oxide block copolymers (e.g. poloxamers), ethoxylated hydrogenated castor oils, and mixtures thereof; preferably poloxamer 407, PEG 40 hydrogenated castor oil and mixtures thereof.

Examples of anionic surfactants are alkyl sulfates and their alkyl-ether derivatives, sarcosinates, isethionates and taurates, such as sodium lauryl sulfate, sodium coconut monoglyceride sulfonate, sodium lauryl sarcosinate, sodium lauryl isethionate, sodium laureth carboxylate and sodium dodecyl benzenesulfonate.

Examples of cationic surfactants are lauryl trimethylammonium chloride, cetyl pyridinium chloride, cetyl trimethylammonium bromide, diisobutylphenoxyethyldimethylbenzylammonium chloride, coconut alkyltrimethylammonium nitrite, cetyl pyridinium fluoride and mixtures thereof.

Examples of amphoteric surfactants are myristyl betaine, palmityl betaine, lauryl betaine, cetyl betaine, stearyl betaine, lauramidopropyl betaine, cocoamidoethyl betaine, cocoamidopropyl betaine and the like.

In a particular embodiment, the amount of surfactant in the compositions of the invention is from 1% w/v to 5% w/v, more preferably from 2% w/v to 3% w/v.

The term "antioxidant" refers to compounds that are capable of inhibiting reactions promoted by oxygen, thus avoiding oxidation and rancidity of the compositions. Examples of antioxidants are panthenol, butylated hydroxyanisole (BHA), butylated hydroxytoluente (BHT), vitamin A, carotenoids, flavonoids, polyphenols, ascorbic acid, herbal antioxidants, clorophyll, melatonin and the like, preferably panthenol. In a particular embodiment, the amount of antioxidant in the compositions of the invention is from 0.05% w/v to 2% w/v, more preferably from 0.1% w/v to 1% w/v.

The term "buffering agent" refers to a compound of mixture of compounds that adjusts and/or maintains the pH of the composition. In particular, the pH of the composition is from 4.0 to 6.0, preferably from 5.0 to 5.5. Examples of suitable buffering agents are citric acid, sodium citrate, sodium benzoate, sodium carbonate, sodium bicarbonate, disodium hydrogen phosphate, sodium dihydrogen phosphate and mixtures thereof, preferably citric acid, sodium citrate and mixtures thereof. In a particular embodiment, the amount of buffering agent in the compositions of the invention is from 0.05% w/v to 2% w/v, more preferably from 0.1% w/v to 0.5% w/v.

The term "thickener" refers to a compound of mixture of compounds that increases the viscosity of the composition above 20 mPa·s when measured at 20° C. Examples of suitable thickeners are hydroxyethyl cellulose (HEC), carboxymethyl cellulose (CMC) and xanthan gum.

The term "flavoring agent" may be added to modify the taste and the aroma of the compositions. The flavoring agents may be natural or synthetic. Examples of flavoring agents are essential oils, flavoring aldehydes, esters, alcohols and others known in the art, such as spearmint oil, cinnamon oil, peppermint oil, clove oil, bay oil, thyme oil, cedar leaf oil, nutmeg oil, sage oil, bitter almond oil, eucalyptus oil, marjoram oil, lemon oil, lime oil, grapefruit oil, orange oil, sassafras oil, artificial vanilla, wintergreen (methyl salicylate), glycyrrhetinic acid and the like, preferably glycyrrhetinic acid. In a particular embodiment, the amount of flavoring agent in the compositions of the invention is from 0.01% w/v to 0.1% w/v, more preferably from 0.01%) w/v to 0.05% w/v.

In a particular embodiment, the aqueous oral rinse composition of the invention comprises:

- from 0.10% w/v to 0.20% w/v of ethyl lauroyl arginate hydrochloride,
- a source of a gluconate anions providing from 15 mM to 100 mM of said anions,
- from 0.05% w/v to 0.5% w/v of Vitamin E or an ester thereof,
- a source of $Zn^{2+}$ cations providing from 1 mM to 50 mM of said cations,
- from 10% w/v to 20% w/v of a humectant,
- from 1% w/v to 5% w/v of a surfactant,
- from 0.05% w/v to 2% w/v of a buffering agent,
- from 0.05% w/v to 2% w/v of an antioxidant, and
- from 0.01% w/v to 0.1% w/v of a flavoring agent.

In another particular embodiment, the aqueous oral rinse composition of the invention comprises:

- from 0.12% w/v to 0.16% w/v of ethyl lauroyl arginate hydrochloride,
- a source of a gluconate anions providing from 17 mM to 100 mM of said anions,
- from 0.10% w/v to 0.30% w/v of Vitamin E or an ester thereof,
- a source of $Zn^{2+}$ cations providing from 3 mM to 50 mM of said cations,
- from 10% w/v to 20% w/v of a humectant,
- from 1% w/v to 5% w/v of a surfactant,
- from 0.05% w/v to 2% w/v of a buffering agent,
- from 0.05% w/v to 2% w/v of an antioxidant, and
- from 0.01% w/v to 0.1% w/v of a flavoring agent.

In another particular embodiment, the aqueous oral rinse composition of the invention comprises:

- from 0.12% w/v to 0.16% w/v of ethyl lauroyl arginate hydrochloride,
- a source of a gluconate anions providing from 19 mM to 100 mM of said anions,
- from 0.15% w/v to 0.25% w/v of Vitamin E or an ester thereof,
- a source of $Zn^{2+}$ cations providing from 5 mM to 50 mM of said cations,
- from 10% w/v to 15% w/v of a humectant,
- from 2% w/v to 3% w/v of a surfactant,
- from 0.1% w/v to 0.5% w/v of a buffering agent,
- from 0.1% w/v to 1% w/v of an antioxidant, and
- from 0.01% w/v to 0.05% w/v of a flavoring agent.

In another particular embodiment, the aqueous oral rinse composition of the invention comprises:

- from 0.13% w/v to 0.15% w/v of ethyl lauroyl arginate hydrochloride,
- a source of gluconate anions providing from 21 mM to 100 mM of said anions,
- from 0.15% w/v to 0.25% w/v of Vitamin E or an ester thereof, and
- a source of $Zn^{2+}$ cations providing from 7 mM to 50 mM of said cations,
- from 10% w/v to 15% w/v of a humectant,
- from 2% w/v to 3% w/v of a surfactant,
- from 0.1% w/v to 0.5% w/v of a buffering agent,
- from 0.1% w/v to 1% w/v of an antioxidant, and
- from 0.01% w/v to 0.05% w/v of a flavoring agent.

In another particular embodiment, the aqueous oral rinse composition of the invention comprises:

- from 0.10% w/v to 0.20% w/v of ethyl lauroyl arginate hydrochloride,
- from 2 mM to 21 mM of sodium gluconate,
- from 5 mM to 50 mM of zinc gluconate,
- from 0.05% w/v to 0.5% w/v of tocopheryl acetate,
- from 10% w/v to 20% w/v of a mixture of glycerin, xylitol and propylene glycol,
- from 1% w/v to 5% w/v of a mixture of poloxamer 407 and PEG40 hydrogenated castor oil,
- from 0.05% w/v to 2% w/v of a mixture of citric acid and sodium citrate,
- from 0.05% w/v to 2% w/v of panthenol, and
- from 0.01% w/v to 0.1% w/v of glycyrrhetinic acid,

wherein the total concentration of gluconate anions is at least 15 mM.

In another particular embodiment, the aqueous oral rinse composition of the invention comprises:

- from 0.12% w/v to 0.16% w/v of ethyl lauroyl arginate hydrochloride,
- from 5 mM to 13 mM of sodium gluconate,
- from 6 mM to 50 mM of zinc gluconate,
- from 0.10% w/v to 0.30% w/v of tocopheryl acetate,
- from 10% w/v to 20% w/v of a mixture of glycerin, xylitol and propylene glycol,
- from 1% w/v to 5% w/v of a mixture of poloxamer 407 and PEG40 hydrogenated castor oil,
- from 0.05% w/v to 2% w/v of a mixture of citric acid and sodium citrate,
- from 0.05% w/v to 2% w/v of panthenol, and
- from 0.01% w/v to 0.1% w/v of glycyrrhetinic acid,

wherein the total concentration of gluconate anions is at least 15 mM..

In another particular embodiment, the aqueous oral rinse composition of the invention comprises:

- from 0.13% w/v to 0.15% w/v of ethyl lauroyl arginate hydrochloride,
- from 7.5 mM to 12.5 mM of sodium gluconate,
- from 6 mM to 50 mM of zinc gluconate,
- from 0.15% w/v to 0.25% w/v of tocopheryl acetate,
- from 10% w/v to 15% w/v of a mixture of glycerin, xylitol and propylene glycol,
- from 2% w/v to 3% w/v of a mixture of poloxamer 407 and PEG40 hydrogenated castor oil,
- from 0.1% w/v to 0.5% w/v of a mixture of citric acid and sodium citrate,
- from 0.1% w/v to 1% w/v of panthenol, and
- from 0.01% w/v to 0.05% w/v of glycyrrhetinic acid,

wherein the total concentration of gluconate anions is at least 15 mM..

The compositions of the invention may also comprise sweeteners to provide a sweet taste. Examples of sweeteners are xylose, ribose, glucose, mannose, lactose, fructose, sucrose, maltose, saccharin salts such as sodium or calcium saccharin salts, cyclamate salts such as sodium cyclamate, aspartame and the like.

The compositions of the invention may also comprise fluoride providing compounds, which are characterized by their ability to release fluoride ions or fluoride containing ions in water. Typical fluoride providing compounds are inorganic fluoride salts such as sodium fluoride, potassium fluoride, ammonium fluoride, cupric fluoride, zinc fluoride, stannic fluoride, stannous fluoride, barium fluoride, sodium hexafluorosilicate, ammonium hexafluorosilicate, sodium fluorozirconate, sodium monofluorophosphate, aluminum mono- and difluorophosphate and fluorinated sodium calcium pyrophosphate; and amine fluorides such as N'-octadecyltrimethylendiamine-N,N,N'-tris(2-ethanol)-dihydrofluoride and 9-octadecenylaminehydrofluoride.

The compositions of the invention may also contain sensitivity reducing agents, such as potassium salts of nitrate and oxalate.

The compositions of the invention may also contain a preservative, such as parabens, potassium sorbate, benzyl alcohol, phenoxyethanol, polyaminopropyl biguanide, caprylic acid, sodium benzoate and cetylpyridinium chloride.

The compositions of the invention may be obtained by mixing the above mentioned ingredients. In particular, the compositions may be prepared by mixing all water soluble components with water, separately mixing the non-water soluble components, then combining the two previous mixtures, preferably under agitation and, if needed, adjusting the pH to from 4.0 to 6.0, preferably from 5.0 to 5.5. More particularly, the compositions may be prepared by:

a) mixing the source of gluconate anions (such as sodium gluconate) and, when present in the compositions, the source of $Zn^{2+}$ anions (such as zinc gluconate), the surfactant(s) (i.e. the water soluble surfactants such as poloxamer 407), the humectant(s) (i.e. the water soluble humectants, such as glycerin and xylitol), the buffering agent(s) (such as sodium citrate and citric acid), and the antioxidant(s) (such as panthenol), with water, preferably with stirring until complete dissolution;

b) to the solution of step a) the vitamin E or an ester thereof (such as $\alpha$-tocopheryl acetate), optionally mixed with the humectant (i.e. the non-water soluble humectants, such as propylene glycol), the surfactant(s) (i.e. the non-water soluble surfactants, such as PEG40 hydrogenated castor oil), flavoring agent(s) (such as glycyrrhetinic acid), and/or further flavoring agents (such as aromas), when present in the compositions of the invention, is then added;

c) ethyl lauroyl arginate hydrochloride is added to the mixture obtained in step b); and

d) if needed, the pH of the mixture obtained in step c) is adjusted to from 4.0 to 6.0, preferably from 5.0 to 5.5 (for example with sodium citrate and/or citric acid).

<u>Use of the aqueous oral rinse compositions</u>

Ethyl lauroyl arginate is known to have the clinical ability to retard the growth of bacteria and, thus, minimize plaque formation, oral infections and dental disease associated therewith.

Plaque is a biofilm or mass of bacteria that grows on surfaces within the oral cavity (mouth). It is a sticky colorless deposit at first but when it forms tartar it is brown or pale yellow and commonly found between the teeth, front of teeth, behind teeth, on chewing surface, along the gumline and below the gumline cervical margins. Plaque can give rise to dental caries and periodontal problems such as gingivitis and periodontitis.

Thus, in a further aspect, the present invention relates to an aqueous oral rinse composition of the invention, as previously defined, for use in inhibiting plaque in an oral cavity.

Alternatively, this aspect may be formulated as a method for inhibiting plaque in an oral cavity comprising contacting a surface of the oral cavity with an aqueous oral rinse composition of the invention.

This aspect may be also formulated as the use of an aqueous oral rinse composition of the invention for the manufacture of a medicament for inhibiting plaque in an oral cavity.

"Inhibiting plaque" refers to prevent the formation of plaque and/or reduce the amount of plaque.

The oral cavity may belong to a human or animal, preferably a human.

For the above purposes, the aqueous oral composition of the invention may be contacted with a surface of the oral cavity. Said surface of the oral cavity may be selected from the group consisting of one or more teeth, surfaces of the gums, surface of the tongue and combinations thereof. The contacting may be performed for any suitable amount of time, such as for less than thirty seconds, for thirty seconds or more, for about thirty seconds, for about forty seconds, for about one minute or for more than one minute. For performing said contacting, the composition of the invention is typically held in the oral cavity passively or swilled around the mouth by contraction of the perioral muscles and/or movement of the head, and may be gargled, and finally spit out.

The amount of composition dosed in the oral cavity to perform the activity is typically from 2-30 ml, preferably from 5-20 ml and most preferable from 10-15 ml.

Use of a combination of a gluconate salt and vitamin E or an ester thereof

As explained above, the authors of the present invention have surprisingly found that combining a gluconate salt and Vitamin E allows obtaining stable low-alcohol or alcohol-free oral rinses comprising ethyl lauroyl arginate hydrochloride.

Thus, in a further aspect the present invention relates to the use of a combination comprising:

- a source of gluconate anions, and
- Vitamin E or an ester thereof,

to enhance the stability of an aqueous oral rinse composition comprising ethyl lauroyl arginate hydrochloride and less than 1% w/v of a $C_1$-$C_4$ monoalcohol, wherein the source of gluconate anions is used in an amount providing at least 15 mM of said anions in the aqueous oral rinse composition.

"Enhance the stability" refers to increase or improve the chemical stability of ethyl lauroyl arginate hydrochloride over time in an aqueous oral rinse composition (i.e. to decrease the degradation of ethyl lauroyl arginate hydrochloride into other chemical products) with respect to a corresponding composition which does not comprise the combination of the gluconate salt and Vitamin E or an ester thereof. The stability of the composition may be determined by quantification of the amount of ethyl lauroyl arginate hydrochloride after storage of the composition under study at a temperature for a period of time and comparing said amount of ethyl lauroyl arginate hydrochloride with the amount of ethyl lauroyl arginate hydrochloride just after preparation of the composition (time=0). This quantification may be performed by using conventional techniques in the art, such as by HPLC (high-performance liquid chromatography), in particular using the HPLC conditions described in the examples. Preferably, a composition is considered to be stable if the concentration of ethyl lauroyl arginate hydrochloride is that of the initial concentration ±15%, more preferably stable if the concentration of ethyl lauroyl arginate hydrochloride is that of the initial concentration ±10%. In an embodiment the compositions of the invention are stable at temperatures up to 40°C, preferably up to 35°C, more preferably up to 30°C.

The aqueous oral rinse composition comprises less than 1% w/v of a $C_1$-$C_4$ monoalcohol, preferably less than 0.5% w/v, more preferably less than 0.01% w/v, even more preferably less than 0.005% w/v. The compositions may also be free from a $C_1$-$C_4$ monoalcohol, i.e. no $C_1$-$C_4$ monoalcohol is present at all. The definition and examples of $C_1$-$C_4$ monoalcohol are the same as described above with respect to the compositions of the invention.

The definition and examples of gluconate salts are the same as described above with respect to the compositions of the invention. Preferably, the gluconate salt is zinc gluconate, sodium gluconate or mixtures thereof.

In said use, the combination comprises a source of gluconate anions providing at least 15 mM of said gluconate anions, preferably at least 17 mM, more preferably at least 19 mM, even more preferably at least 21 mM, still more preferably about 24 mM. In said use, the combination comprises a source of gluconate anions providing from 15 mM to 100 mM of said gluconate anions, more preferably from 17 mM to 100 mM, even more preferably from 19 mM to 100 mM, still more preferably from 21 mM to 100 mM. As explained above, if several sources of gluconate anions are present, the amount of gluconate anions refers to the sum of all of the gluconate anions present in the composition.

The definition and examples of Vitamin E and esters thereof are the same as described above with respect to the compositions of the invention. In a particular embodiment, the Vitamin E or ester thereof is tocopheryl acetate (including stereoisomers thereof) and mixtures thereof, more preferably $\alpha$-tocopheryl acetate.

Preferably, the combination comprises from 0.01% w/v to 1% w/v of Vitamin E or an ester thereof, more preferably from 0.05% w/v to 0.5% w/v, even more preferably from 0.1% w/v to 0.3% w/v, even more preferably from 0.15% w/v to 0.25% w/v, still more preferably about 0.2% w/v.

In the above mentioned use, preferably the combination comprises a source of gluconate anions providing

from 15 mM to 100 mM of gluconate anions and from 0.05% w/v to 0.5% w/v of Vitamin E or an ester thereof.

In the above mentioned use, preferably the combination further comprises a source of $Zn^{2+}$ cations. The definition and sources of $Zn^{2+}$ cations are the same as defined above with respect of the composition of the invention. Preferably the source of $Zn^{2+}$ cations is zinc gluconate

Preferably, the source of $Zn^{2+}$ cations is present in an amount to provide at least 1 mM, preferably at least 3 mM, more preferably at least 5 mM, even more preferably at least 7 mM, still more preferably at least 7.4 mM. Preferably the source of $Zn^{2+}$ cations is present in an amount to provide a range of from 1 mM to 50 mM of $Zn^{2+}$, more preferably from 3 mM to 50 mM, even more preferably from 5 mM to 50 mM, even more preferably from 7 mM to 50 mM, still more preferably from 7.4 mM to 50 mM.

In a particular embodiment, the amount of ethyl lauroyl arginate hydrochloride present in the aqueous oral rinse composition to be stabilized is from 0.01% w/v to 1% w/v, preferably from 0.05% w/v to 0.5% w/v, even more preferably from 0.10% w/v to 0.20% w/v, still more preferably from 0.12% w/v to 0.16% w/v, still more preferably from 0.13% w/v to 0.15% w/v.

As defined above, the term "aqueous" refers to compositions comprising water, preferably at least 50% w/v, preferably at least 60% w/v, more preferably at least 70% w/v, even more preferably at least 75% w/v, still more preferably at least 80% w/v of water.

The aqueous oral rinse compositions may comprise further ingredients as described above, e.g. humectants, surfactants, antioxidants, buffering agents and flavoring agents.

## Examples

Compositions

100 ml of aqueous oral rinse compositions were prepared by adding poloxamer 407, glycerin, xylitol, sodium citrate, citric acid, zinc gluconate (when present in the composition), sodium gluconate and panthenol to water and stirring until complete dissolution. To this solution, a premix of propylene glycol, PEG40 hydrogenated castor oil, glycyrrhetinic acid, and α-tocopheryl acetate (when present in the composition) was added. Finally ethyl lauroyl arginate hydrochloride was added and the pH was adjusted to 5.3 + 0.5 (with sodium citrate and/or citric acid as needed). The ingredients and their amounts (expressed as % w/v, unless otherwise stated) of each composition is provided in the table below. Example 1 is a composition according to the present invention. Examples 2 and 3 are comparative examples.

| *Ingredient* | *Example 1* | *Comparative example 2* | *Comparative example 3* |
|---|---|---|---|
| Ethyl lauroyl arginate hydrochloride (20% in glycerin) | 0.710 | 0.710 | 0.710 |
| Zinc gluconate | 0.340(1) | - | 0.340(1) |
| Xylitol | 1.000 | 1.000 | 1.000 |
| Glycyrrhetinic acid | 0.030 | 0.030 | 0.030 |
| α-Tocopheryl acetate | 0.200 | 0.200 | - |
| Panthenol | 0.500 | 0.500 | 0.500 |
| Poloxamer 407 | 0.200 | 0.200 | 0.200 |
| Propylene glycol | 7.000 | 7.000 | 7.000 |
| Glycerin | 5.000 | 5.000 | 5.000 |
| PEG40 hydrogenated castor oil | 2.500 | 2.500 | 2.500 |
| Citric acid | 0.040 | 0.100 | 0.040 |
| Sodium citrate | 0.325 | 0.325 | 0.325 |
| Sodium gluconate | 0.200(2) | 0.200(2) | 0.200(2) |
| Water | *q.s.* 100 ml | *q.s.* 100 ml | *q.s.* 100 ml |
| pH | 5.33 | 5.35 | 5.18 |
| (1) equivalent to 14.92 mM of gluconate anions and 7.46 mM $Zn^{2+}$ cations<br>(2) equivalent to 9.17 mM of gluconate anions | | | |

Method for determining the concentration of ethyl lauroyl arginate hydrochloride

Equipment: Chromatograph HPLC Chromaster with Diode Array Detector
Column: Acclaim Surfactant (Special Silica-based C18 column for separation of surfactants) 5μm (15 x 0.46 cm) DIONEX or equivalent
Mobil phase: (A) Acetonitrile, (B) Acetate Buffer (0.1 M, pH 5.4)

| Gradient: | Time (min) | %A | %B | Flow (ml/min) |
|---|---|---|---|---|
| | 0.0 | 36 | 64 | 1.5 |
| | 11.0 | 36 | 64 | 1.5 |
| | 11.5 | 50 | 50 | 1.5 |
| | 21.5 | 50 | 50 | 1.5 |
| | 22.0 | 36 | 64 | 1.5 |
| | 30.0 | 36 | 64 | 1.5 |

Detection: UV at 220 nm
T (oven): 30°C

Stability studies

The compositions of Example 1, Comparative example 2 and Comparative example 3 were stored in closed glass vials (20 ml) for 5 days at different temperatures, namely 5 °C, 50 °C, 60°C and 70 °C. After these 5 days of storage, the concentration of ethyl lauroyl arginate hydrochloride was determined using the method described above.

For each composition, the concentration values thus obtained were used for calculating the reaction constant $k$ (in $sec^{-1}$) of degradation of ethyl lauroyl arginate hydrochloride at each tested temperature $T$ by using equation 1:

$$k = \frac{2.303}{t} \log \frac{a}{a-x} \qquad \textit{Equation 1}$$

wherein $a$ is the initial concentration of ethyl lauroyl arginate hydrochloride in moles (i.e. at $t$=0), $a$-$x$ is the final concentration of ethyl lauroyl arginate hydrochloride in moles (in the present assay after storage for 5 days), and $t$ is the time in seconds.

For each composition, the calculated $k$ values (one for each temperature) were then used to correlate $k$ with $T$ (temperature in °K) by using equation 2:

$$\log k = -\frac{E_a}{4.57}\frac{1}{T} + \log z \qquad \textit{Equation 2}$$

wherein $E_a$ is the activation energy (in cal/mol) and z is the frequency factor (in $sec^{-1}$) ($k$ and $T$ have been previously defined).

For each composition a straight line is obtained by representing log $k$ vs 1/$T$, said straight line has $-E_a/4.57$ as the slope and log $z$ as the y-intercept (according to equation 2). This straight line was then used to predict the $k$ values for each composition at other temperatures, in particular at 25 °C, 30 °C and 40 °C. These $k$ values were then used to predict the final concentration of ethyl lauroyl arginate hydrochloride in each composition after storage for a time $t$ at the temperature of interest (in particular at 25 °C, 30 °C and 40 °C) by using equation 1.

The results of ethyl lauroyl arginate hydrochloride degradation for the composition of Example 1, of Comparative example 2 and of Comparative example 3 are shown in Figures 1, 2 and 3, respectively. A composition is considered to be stable if the predicted concentration of ethyl lauroyl arginate hydrochloride after storage falls within the range of the initial concentration ±15%, preferably within the range of the initial concentration ±10%. As it can be seen in Figures 1-3, in the composition of Example 1 the concentration of ethyl lauroyl arginate hydrochloride remains stable at 25 °C, 30 °C and 40 °C after 36 months of storage, whereas in compositions of Comparative example 2 and Comparative example 3 the concentration of ethyl lauroyl arginate hydrochloride decreases with time beyond the limits of ±9% of the initial concentration of ethyl lauroyl arginate hydrochloride.

## Claims

1. Aqueous oral rinse composition comprising ethyl lauroyl arginate hydrochloride, a source of gluconate anions, and Vitamin E or an ester thereof, wherein the composition comprises less than 1% w/v of a $C_1$-$C_4$ monoalcohol and wherein the source of gluconate anions is used in an amount providing at least 15 mM of said anions in the aqueous oral rinse composition.

2. Aqueous oral rinse composition according to claim 1, which comprises from 0.10% w/v to 0.20% w/v of ethyl lauroyl arginate hydrochloride,

3. Aqueous oral rinse composition according to any one of the preceding claims, which comprises a source of gluconate anions providing from 15 mM to 100 mM of gluconate anions.

4. Aqueous oral rinse composition according to any one of the preceding claims, which comprises from 0.05% w/v to 0.5% w/v of Vitamin E or an ester thereof.

5. Aqueous oral rinse composition according to any one of the preceding claims, further comprising a source of $Zn^{2+}$ cations.

6. Aqueous oral rinse composition according to claim 5, wherein the source of $Zn^{2+}$ cations is present in an amount providing a concentration of at least 1 mM, preferably from 1 mM to 50 mM of $Zn^{2+}$ cations.

7. Aqueous oral rinse composition according to any one of the preceding claims, which comprises zinc gluconate and/or sodium gluconate.

8. Aqueous oral rinse composition according to any one of the preceding claim, wherein the Vitamin E or an ester thereof is tocopheryl acetate.

9. Aqueous oral rinse composition according to any one of the preceding claims further comprising one or more ingredients selected from the group consisting of humectants, surfactants, antioxidants, buffering agents and flavoring agents.

10. Aqueous oral rinse composition as defined in any one of claims 1 to 9 for use in inhibiting plaque in an oral cavity.

11. Use of a combination comprising:

    - a source of gluconate anions , and
    - Vitamin E or an ester thereof,

    to enhance the stability of an aqueous oral rinse composition comprising ethyl lauroyl arginate hydrochloride and less than 1% w/v of a $C_1$-$C_4$ monoalcohol, wherein the source of gluconate anions is used in an amount providing at least 15 mM of said anions in the aqueous oral rinse composition.

12. Use according to claim 11, wherein the combination comprises a source of gluconate anions providing from 15 mM to 100 mM of said anions and from 0.05% w/v to 0.5% w/v of Vitamin E or an ester thereof.

13. Use according to any one of claims 11 or 12, wherein the combination further comprises a source of $Zn^{2+}$ cations in an amount to provide, preferably at least 0.1 mM of $Zn^{2+}$, more preferably from 0.1 mM to 50 mM of $Zn^{2+}$ cations to the oral rinse composition.

14. Use according to any one of claims 11 to 13, wherein the combination comprises zinc gluconate and/or sodium gluconate.

15. Use according to any one of claims 11 to 14, wherein the Vitamin E or an ester thereof is tocopheryl acetate.

**Patentansprüche**

1. Wässrige Mundspülungszusammensetzung, umfassend Ethyllauroylarginathydrochlorid, eine Quelle von Gluconatanionen, und Vitamin E oder einen Ester davon, wobei die Zusammensetzung weniger als 1% w/v eines $C_1$--$C_4$--Monoalkohols umfasst, und wobei die Quelle von Gluconatanionen in einer Menge verwendet wird, die mindestens 15mM der besagten Anionen in der wässrigen Mundspülungszusammensetzung bereitstellt.

2. Wässrige Mundspülungszusammensetzung nach Anspruch 1, die 0,10% w/v bis 0,20% w/v Ethyllauroylarginathydrochlorid umfasst.

3. Wässrige Mundspülungszusammensetzung nach einem der vorstehenden Ansprüche, die eine Quelle von Gluconatanionen umfasst, die 15mM bis 100mM Gluconatanionen bereitstellt.

4. Wässrige Mundspülungszusammensetzung nach einem der vorstehenden Ansprüche, die 0,05% w/v bis 0,5% w/v Vitamin E oder einen Ester davon umfasst.

5. Wässrige Mundspülungszusammensetzung nach einem der vorstehenden Ansprüche, ferner umfassend eine Quelle für $Zn^{2+}$-Kationen.

6. Wässrige Mundspülungszusammensetzung nach Anspruch 5, wobei die Quelle von $Zn^{2+}$-Kationen in einer Menge vorhanden ist, die eine Konzentration von mindestens 1mM, vorzugsweise von 1mM bis 50mM von $Zn^{2+}$-Kationen bereitstellt.

7. Wässrige Mundspülungszusammensetzung nach einem der vorstehenden Ansprüche, die Zinkgluconat und/oder Natriumgluconat umfasst.

8. Wässrige Mundspülungszusammensetzung nach einem der vorstehenden Ansprüche, wobei das Vitamin E oder ein Ester davon Tocopherylacetat ist.

9. Wässrige Mundspülungszusammensetzung nach einem der vorstehenden Ansprüche, ferner umfassend einen oder mehrere Inhaltsstoffe, ausgewählt aus der Gruppe bestehend aus Feuchthaltemitteln, Tensiden, Antioxidantien, Puffermitteln und Aromastoffen.

10. Wässrige Mundspülungszusammensetzung wie nach einem der Ansprüche 1 bis 9 definiert zur Verwendung bei der Hemmung von Plaque in einer Mundhöhle.

11. Verwendung einer Kombination, umfassend:

    - eine Quelle von Gluconatanionen, und
    - Vitamin E oder ein Ester davon,

    um die Stabilität einer wässrigen Mundspülzusammensetzung zu erhöhen, die Ethyllauroyarginathydrochlorid und weniger als 1% w/v eines $C_1$-$C_4$-Monoalkohols umfasst, wobei die Quelle von Gluconatanionen in einer Menge verwendet wird, die mindestens 15mM der besagten Anionen in der wässrigen Mundspülzusammensetzung bereitstellt.

12. Verwendung nach Anspruch 11, wobei die Kombination eine Quelle von Gluconatanionen umfasst, die 15mM bis 100mM der besagten Anionen und 0,05% w/v bis 0,5% w/v Vitamin E oder einen Ester davon bereitstellt.

13. Verwendung nach einem der Ansprüche 11 oder 12, wobei die Kombination ferner eine Quelle von $Zn^{2+}$-Kationen in einer Menge umfasst, die vorzugsweise mindestens 0,1mM an $Zn^{2+}$, noch bevorzugter 0,1mM bis 50mM an $Zn^{2+}$-Kationen in der Mundspülzusammensetzung bereitstellt.

14. Verwendung nach einem der Ansprüche 11 bis 13, wobei die Kombination Zinkgluconat und/oder Natriumgluconat umfasst.

15. Verwendung nach einem der Ansprüche 11 bis 14, wobei das Vitamin E oder ein Ester davon Tocopherylacetat ist.

## Revendications

1. Composition aqueuse de rinçage oral comprenant du chlorhydrate d'éthyle lauroyl arginate, une source d'anions gluconate et de la vitamine E ou un ester de celle-ci, la composition comprenant moins de 1 % p/v d'un monoalcool en $C_1$-$C_4$ et la source d'anions gluconate étant utilisée en une quantité fournissant au moins 15 mM desdits anions dans la composition aqueuse de rinçage oral.

2. Composition aqueuse de rinçage oral selon la revendication 1, qui comprend de 0,10 % p/v à 0,20 % p/v de chlorhydrate d'éthyle lauroyl arginate.

3. Composition aqueuse de rinçage oral selon l'une quelconque des revendications précédentes, qui comprend une source d'anions gluconates fournissant de 15 mM à 100 mM de gluconates anions.

4. Composition aqueuse de rinçage oral selon l'une quelconque des revendications précédentes, qui comprend de 0,05 % p/v à 0,5 % p/v de vitamine E ou d'un ester de celle-ci.

5. Composition aqueuse de rinçage oral selon l'une quelconque des revendications précédentes, comprenant en outre une source de cations $Zn^{2+}$.

6. Composition aqueuse de rinçage oral selon la revendication 5, dans laquelle la source de cations $Zn^{2+}$ est présente en une quantité fournissant une concentration d'au moins 1 mM, de préférence de 1 mM à 50 mM de cations $Zn^{2+}$.

7. Composition aqueuse de rinçage oral selon l'une quelconque des revendications précédentes, qui comprend du gluconate de zinc et/ou du gluconate de sodium.

8. Composition aqueuse de rinçage oral selon l'une quelconque des revendications précédentes, dans laquelle la vitamine E ou un ester de celle-ci est l'acétate de tocophérol.

9. Composition aqueuse de rinçage oral selon l'une quelconque des revendications précédentes comprenant en outre un ou plusieurs ingrédients choisis dans le groupe constitué par les humectants, les surfactants, les antioxydants, les agents tampon et les agents aromatisants.

10. Composition aqueuse de rinçage oral selon l'une quelconque des revendications 1 à 9 pour une utilisation dans l'inhibition de la plaque dans une cavité buccale.

11. Utilisation d'une combinaison comprenant :

    - une source d'anions gluconate, et
    - de la vitamine E ou un ester de celle-ci,

    pour améliorer la stabilité d'une composition aqueuse de rinçage oral comprenant du chlorhydrate d'éthyle lauroyle arginate et moins de 1 % p/v d'un monoalcool en $C_1$-$C_4$, la source des anions gluconate étant utilisée en une quantité fournissant au moins 15 mM desdits anions dans la composition aqueuse de rinçage oral.

12. Utilisation selon la revendication 11, dans laquelle la combinaison comprend une source d'anions gluconates fournissant de 15 mM à 100 mM desdits anions et de 0,05 % p/v à 0,5 % p/v de vitamine E ou d'un ester de celle-ci.

13. Utilisation selon l'une quelconque des revendications 11 ou 12, dans laquelle la combinaison comprend en outre une source de cations $Zn^{2+}$ en une quantité permettant de fournir à la composition de rinçage oral de préférence au moins 0,1 mM de $Zn^{2+}$, et de façon encore préférée, de 0,1 mM à 50 mM de cations $Zn^{2+}$,.

14. Utilisation selon l'une quelconque des revendications 11 à 13, dans laquelle la combinaison comprend du gluconate de zinc et/ou du gluconate de sodium.

15. Utilisation selon l'une quelconque des revendications 11 à 14, dans laquelle la vitamine E ou un ester de celle-ci est l'acétate de tocophérol.

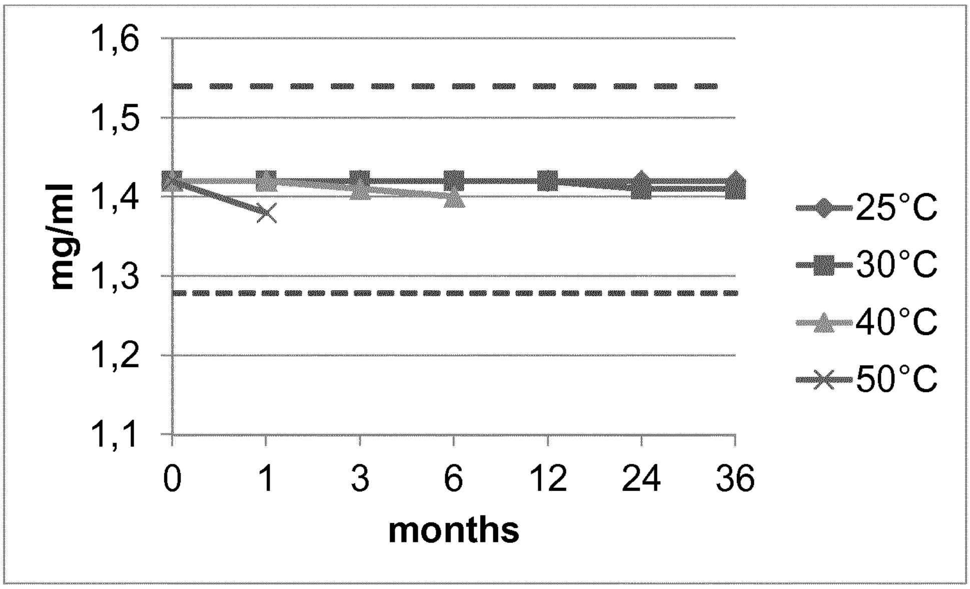
1,6
1,5
1,4
1,3
1,2
1,1
mg/ml
0
1
3
6
12
24
36
months
25°C
30°C
40°C
50°C

FIG. 1

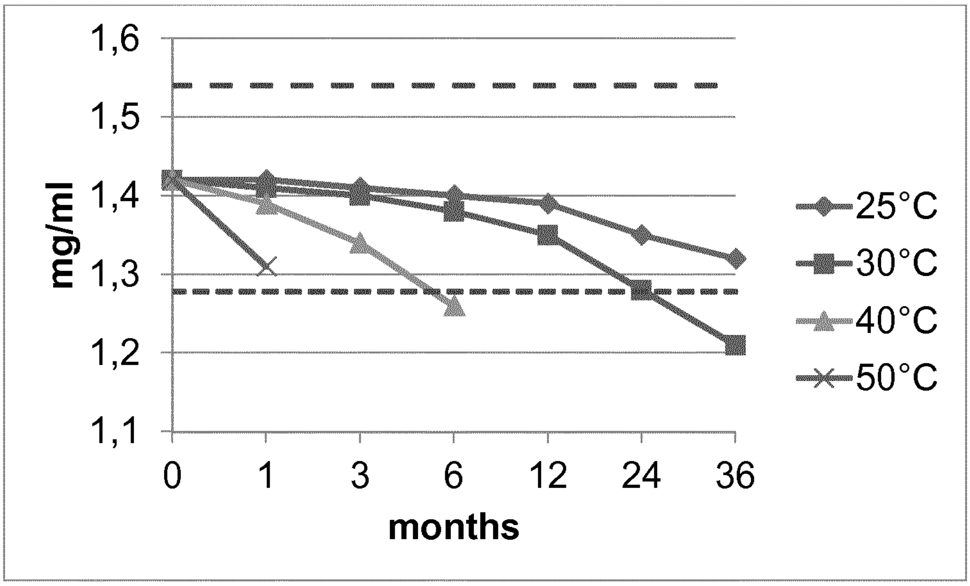
1,6
1,5
1,4
1,3
1,2
1,1
mg/ml
0
1
3
6
12
24
36
months
25°C
30°C
40°C
50°C

FIG. 2

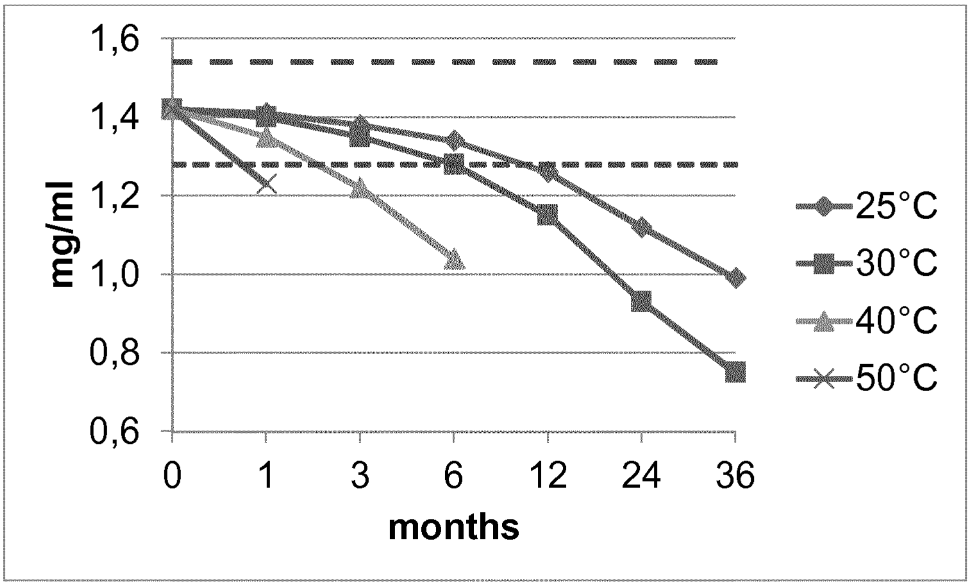


FIG. 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5874068 A
- US 20100330136 A1
- WO 2016077464 A1
- WO 2007011552 A2